# EUROPEAN PATENT APPLICATION

(11) **EP 2 434 858 A1**
(43) Date of publication of application: **28.03.2012**
(21) Application number: 10178798.4
(22) Date of filing: 23.09.2010
(51) Int. Cl.: H05K 9/00, A61N 1/16

(54) **Shielding device for attenuating electromagnetic fields**

(71) Applicant: Lim, Jes Tyng-Yee, Sydney, NSW 2009 (AU)
(72) Inventor: Lim, Jes Tyng-Yee, Sydney, NSW 2009 (AU)
(74) Representative: Winter, Brandl, Fürniss, Hübner, Röss, Kaiser, Polte - Partnerschaft

(57) **Abstract**

Shielding device for attenuating electromagnetic fields in buildings, comprising:
a) an AC power source;
b) at least two frequency attenuation units (1), each comprising a conductor (2), being coaxially arranged in a glass or metal tube (3), wherein said conductor (2) is wrapped in an insulating material (4), and said conductor (2) is electrically connected to said power source; and
c) at least one metal plate (5), provided between two of said frequency units (1).

## Description

The present invention relates to a shielding device for attenuating electromagnetic fields in buildings in accordance with the preamble of claim 1.

In the outer environment, in modern homes, offices, industrial working places, as well as in cars, human beings are exposed to electromagnetic fields of different sources, various strengths and a huge manifold of frequencies, which often is also referred to as "electrosmog".

Technically, the term "electromagnetic field" (EMF) generally refers to all fields throughout the electromagnetic spectrum, however, for the purpose of the present invention is understood as not comprising ionizing radiation such as α-, p- or y-rays as emitted by radionuclides. Moreover, the term EMF for the purpose of the present invention particularly refers to low-frequency radiation fields, i.e. such fields below 300 Hertz (Hz), and in particular to those fields in the 50 to 60 Hz range, which are also known as power-frequency EMFs. However, the term EMF also refers to high-frequency radiation in the frequency range from 1 MHz to 300 GHz, which are used by a number of technologies such as in radio transmitter, microwave ovens, mobile phones and wireless LAN networks as well as Bluetooth interfaces and remote control devices.

As a type of non-ionizing radiation, EMFs in this range do not have sufficient energy to remove an electron from an atom or molecule, but generally transfer thermal energy to other particles. Power-frequency EMFs are those generated by electric power delivery systems. Recently, there has been the greatest public concern and research about possible adverse human health effects.

From the physical point of view, power-frequency EMFs have two components: electric fields and magnetic fields. The electric fields are generated from wave like motion of charges, in particular in alternating current power lines. The magnetic fields, on the other hand, are generated from the actual electrical current, or the flow of electricity. Thus, when a standard household electric light is plugged into a live electrical socket, but turned off, it generates only an electric field. Once turned on, it generates both electric and magnetic fields, since the voltage is still present but current is now flowing. The size of a magnetic field increases as the amount of current flow increases, as the size of the source increases, and as one gets nearer to the source. Adverse health effects from acute exposures include shocks, burns, and death (by electrocution). Generally, from chronic exposures - according to today's knowledge - only magnetic fields have shown associations with adverse human health effects in epidemiologic studies, rather than electrical fields.

Until the late 1970s, it had been assumed that power frequency EMFs were too weak, or had too little energy, to cause biologic effects. Then, in 1979, Nancy Wertheimer and Ed Leeper published an epidemiologic study that showed that children in Denver, Colorado, who died of cancer, particularly leukemia, were more likely to live in houses with higher EMF exposures than children of similar ages living in the same neighborhoods (Wertheimer N, Leeper, E: Am J Epidemiol. (1979), Mar;109(3):273-84: "Electrical wiring configurations and childhood cancer").

According to Wertheimer et al., an excess of electrical wiring configurations suggestive of high current-flow was noted in Colorado in 1976--1977 near the homes of children who developed cancer, as compared to the homes of control children. The finding was strongest for children who had spent their entire lives at the same address, and it appeared to be dose-related. It did not seem to be an artifact of neighborhood, street congestion, social class, or family structure. The reason for the correlation is uncertain; possible effects of current in the water pipes or of AC magnetic fields are suggested.

The paper of Wertheimer et al. set off a flurry of research seeking to determine whether those with high exposures to magnetic fields at home or in the workplace might be at greater risk of getting and/or dying of cancer than those with lower exposures. In general, the most consistent and compelling cancer data are found in the studies of children exposed to EMFs in their homes. While there is still much controversy, the most well-conducted studies suggest a slight excess of childhood leukemia, particularly among those most highly exposed. In workers, the data show weaker support for excess leukemia and brain cancers. A variety of other adverse effects have also been investigated, including adverse reproductive outcomes, neurodegenerative diseases, and cardiac abnormalities. However, there are fewer of these studies, the studies have more methodologic limitations, and the results are more uncertain.

There are some methods for reducing exposure to magnetic fields. For large sources, such as outdoor power lines, sometimes similar strength currents can be set up to run in opposite direction to each other so that they cancel out most of the magnetic fields. For smaller sources, such as appliances (e.g. electric blankets, hair dryers, televisions), the magnetic fields fall off rapidly with the square of distance, so increasing the distance from a source rapidly reduces exposure.

Furthermore, a typical and well accepted shielding is achieved by shielding meshes around sources of EMFs. This principle is known, for example in antenna cables, where the signal guiding inner conductor is shielded by a metal mesh or metal foil wrapped around an insulator of the signal guiding inner conductor in order to prevent noise radiation or other signals from penetrating the signal guiding conductor and finally blurring the signal.

With respect to low-frequency EMFs and adverse effects to human beings it is referred to the following literature:
National Research Council (1997). Possible Health Effects of Exposure to Residential Electric and Magnetic Fields. Washington, DC: National Academy Press.
NIEHS Working Group (1998). Assessment of Health Effects from Exposure to Power-Line Frequency Electric and Magnetic Fields, ed. C. J. Portier and M. S. Wolfe. NIH Publication No. 98-3981. Research Triangle Park, NC: National Institute of Environmental Health Sciences.
Reilly, J. P. (1998). Applied Bioelectricity. New York: Springer-Verlag.

With respect to high-frequency EMF, it is referred to Repacholi, M.H., Toxicology Letters 120 (2001), 323-331: "Health rrisks from the use of mobile phones":

Concern continues about exposure to radiofrequency (RF) fields from sources used for mobile telecommunications, radars, radio and television broadcast, medical and industrial applications. Much of this concern arises because new technologies are introduced without provision of public information about their nature or discussion of the debate within the scientific community about possible health consequences. In the meantime mobile phone use has increased dramatically with falling costs. Industry sources suggest that there were over one billion users worldwide by 2005, far exceeding telephone use via fixed-lines.

Of particular concern to the World Health Organization (WHO) is the fact that, if any adverse health effect is established from mobile phone use, it will be a global concern because developing countries are establishing this technology in preference to the more-expensive fixed line systems. Thus, even a small impact on health could have a major public health consequence.

The output power from the antennas of digital mobile telephones is much lower than the earlier technology analogue models. However, RF emissions from the base stations that communicate between the mobile phones and the networks are orders of magnitude smaller than from handsets. Yet the key concern of the public has been the apparently ad hoc erection of base stations in their living environment without adequate consultation.

To compound the problem, the media sees EMFs as a newsworthy issue so that newly published scientific research is sensationally reported without qualification from the results of previous studies. To address these concerns, WHO established the International EMF Project in 1996 and is collaborating with eight international organizations, seven WHO collaborating institutions and over 45 national authorities. The International EMF Project, in collaboration with the International Commission on Non-Ionizing Radiation Protection (ICNIRP), has completed initial international scientific reviews of possible health effects of exposure to EMF. These reviews provide interim conclusions on health hazards from exposure to EMF and gaps in knowledge requiring further research before better health risk assessments could be made by WHO. Results of the RF review covering the frequency range 10 MHz to 300 GHz have been published (Repacholi, M.H., Bioelectromagnetics 19 (1998), 1-19: "Low-level exposure to radiofrequency electromagnetic fields: health effects and research needs").

Sufficient concern in Europe was voiced e.g. in the UK that the Minister for Public Health established an Independent Expert Group on Mobile Phones (IEGMP) 'To consider present concerns about the possible health effects from the use of mobile phones, base stations and transmitters, to conduct a rigorous assessment of existing research and to give advice based on the present state of knowledge. This group has published its report (IEGMP, 2000) and the results are available on its web site: http://www.iegmp.org.uk.

As adverse effects Repacholi (2001) mentions the so-called "microwave hearing effect" of RF fields with carrier frequencies between 200 MHz and 6.5 GHz. Upon exposure to such RF fields, people with normal hearing perceive these RF pulses as a kind of hearing. The sound has been variously described as a buzzing, clicking, hissing or popping sound, depending on modulation characteristics. Prolonged or repeated exposure may be stressful.

Furthermore, the retina, iris and corneal endothelium of the primate eye were reported to be susceptible to low-level RF fields, particularly when pulsed. Various degenerative changes in light sensitive cells in the retina were reported at specific energies per pulse (10 ms pulses at 100 p.p.s.), as low as 2.6 mJ/kg after the application of a drug used in glaucoma treatment. However, these results could not be replicated.

However, by far the greatest public concern has been that exposure to low-level RF fields may cause cancer. Of the epidemiological studies addressing possible links between RF exposure and excess risk of cancer, some positive findings were reported for leukaemia and brain tumours. Overall, the results are inconclusive and do not support the hypothesis that exposure to RF fields causes or influences cancer.

Nevertheless, according to the current state of scientific investigations, adverse effects of EMFs in the low and high-frequency range, including cancer causing or at least cancer promotion cannot be excluded as a result of electro smog exposure.

In order to prevent or attenuate EMFs, a number different approaches have been described in the prior art.

Starting with the above mentioned Faraday's cage type shielding meshes, the art further has developed sophisticated devices for lowering EMF exposure to human beings.

German Utility Model DE 20312045 (U1) describes a medium containing feldspar, raw limestone, quartz, clay, bog soil, barium sulfate, Artemisia vulgaris, Crepis biennis and water, with 33% oxygen, used for screening from radiation from electronic products, inductive and capacitive radiation and transmitter frequencies, especially in the range 400 MHz to 2.0 GHz.

Additionally, German Utility Model DE 202006015409 (U1) discloses an electrosmog removal bed installation having polyurethane fabric covers enclosing a breathable pad and silver coated fabric layer fixed to an edge strip with press stud contacts and connection cable to an earth connector.

Furthermore, US 7741561 B2 and W02007/082326 A1 disclose an electrosmog shield, wherein in combination with an alternating-current-carrying electric power cable and a guide holding the cable is described. The guide is constructed such that electrical or magnetic radiation emitted by the cable can pass through the guide, an electrosmog shield has a channel formed of a material capable of blocking low-frequency electrical and magnetic radiation and dimensioned to fit with the conduit.

However, most of the prior art devices are either designed for a highly specific purpose such as bed or power-outlet, or are inconvenient to handle such as compositions. Furthermore, a disadvantage is the lack of mobility in fixed installations such as the device of US 7741561 B2 and W02007/082326 A1.

Thus, starting from the closest prior art of US 7741561 B2 and W02007/082326 A1 it was the problem of the present application to provide an alternative easy to handle shielding device, preferably for use inside a building to attenuate electromagnetic fields of different sources.

This problem is solved by a shielding device in accordance with claim 1.

In particular, the present invention relates to a shielding device for attenuating electromagnetic fields in buildings characterized by comprising:
a) an AC power source;
b) at least two frequency attenuation units, each comprising a conductor, being coaxially arranged in a glass or metal tube, wherein said conductor is wrapped in an insulating material, and said conductor is electrically connected to said power source; and
c) at least one metal plate, provided between two of said frequency units (1).

When connected to an alternating-current power source, all parts of the device are acting together, yielding in an attenuation of electromagnetic fields in the room where the shielding device is operated. Although, an exact physical or mechanistic explanation cannot be provided at the moment, the inventor has carried out a number of empirical and practical tests to confirm the effects of the shielding device of the present invention of attenuating the EMFs of a daily home and office environment.

For example, it is known from the experimental scientific work of Dr. Masaru Emoto (vgl. Emoto, M: "Die Botschaft des Wassers", KOHA-Verlag, Burgrain, 2002) that frozen distilled water drops of appr. 100 µl volume only rarely yield to crystals if treated by freezing on a hydrophobic surface at -30°C for one hour, and then watching the tip of the pyramidally shaped frozen drops by an optical microscope for crystal formation (so-called "Emoto-method").

However, when exposing distilled water only for 5 minutes to the shielding device of the present invention, significant crystal formation in the Emoto-method can be observed. Such crystal formation is an indication for an energetic interaction with the shielding device of the present invention and the EMFs from different sources and the distilled water. In particular, the crystal formation in the distilled water samples is a kind of measure for crystal growth being undisturbed by electromagnetic fields, and hence, indirect evidence for the effectivity of the shielding device of the present invention.

At the moment, there are several bio-feedback machines by trade names "SCIO", "ETASCAN" and "I-HEALTH" which are used by healthcare workers, health therapists and medical doctors in Germany, Britain, Austria, Russia and many countries in the European Union to carry out body health and nutrients analysis on individuals. These bio-feedback machines can also be used to test and make prove on the effectiveness of the present invention.

Furthermore, preferred embodiments of the present invention include:

A shielding device wherein said conductors are selected from the group consisting of: graphite, aluminum, titanium, vanadium, chromium, molybdenum, iron, cobalt, nickel, zinc, copper, silver, gold, platinum, palladium.

The selection of different metals for the conductors have the advantage that the conductors can be adapted to different needs of shielding. A particular suitable conductor is one made of copper.

It is preferred that the shielding device's glass tubes are selected from the group consisting of: alkali silicate glass, earth alkaline glass, quartz, borosilicate glass.

Again, the choice of materials enables a number of fine adjustments to certain kinds of EMFs. In practice, it has turned out that tubes made of borosilicate glass gave the best results in attenuation of the EMFs concerned.

However, depending on the kind of EMFs, it also might be advantageous to use metal tubes in a shielding device according to the present invention. For the purpose of fine tuning the shielding device, said metal tubes may be made of a metal being selected from the group consisting of: aluminum, titanium, vanadium, chromium, molybdenum, iron, cobalt, nickel, zinc, copper, silver, gold, platinum, palladium; and alloys thereof.

In a further preferred embodiment of the shielding device, the insulating material which is wrapped around the conductor, is a bound or unbound, woven or unwoven mineral wool or glass fibre wool, in particular a woven glass fibre mat.

For enhancing shielding and attenuation effects, the shielding device according to the present invention might bear vertically or horizontally arranged patterns on the surfaces of the glass tubes of said frequency attenuation units. A particular preferred pattern used in the shielding device is an S-shaped pattern.

Typically, said patterns are created by grinding or etching grooves, or by applying coatings made of graphite or metallic base paints showing a high conductivity and/or deflection properties for electromagnetic fields. In particular, the latter has the advantage that the shielding effects may be enhanced.

For practical purposes, in particular for an adaptation to the volume to be shielded and the strength of the EMFs, the inventor has found out that it is advisable that the glass tubes of the frequency attenuation units have a diameter of 0.25 cm to 3 m and of various thickness according to the requirement of each project.

If desirable, up to 50 frequency attenuation units can be bundled together to be able to be operated in large scale application.

An important part of the shielding device of the present invention is the (preferably programmed) metal plate positioned between two frequency attenuation units or a larger (also preferably programmed) metal plate which can be placed inside over the two frequency attenuation units according to requirement. This plate can be made from a number of metals. Usually, it is made from highly pure aluminum, but also precious metals such as gold, platinum and iridium or alloys thereof may be used.

In particular, said metal plate between said frequency attenuation units (1) is made of a metal being selected from the group consisting of: aluminum, titanium, vanadium, chromium, molybdenum, iron, cobalt, nickel, zinc, copper, silver, gold, platinum, iridium, palladium; and alloys thereof.

As a further preferred embodiment of the present invention, a metal plate of the Shen-Ion HarmoNIK (TM) type can be used. This has the advantage, that the multiple programmed frequencies information in the Shen-Ion HarmoNIK(TM) metal plate not only harmonise, balance and alter the electrons and magnetic frequencies, but also are programmed to attract and to absorb free electrons, tachyons and static electricity from the air to supplement, improve and save electricity consumption.

It is preferred that such parts of the conductors which are located outside the frequency attenuation units, are supported by at least one cradle in order to keep the conductors in position. In a further preferred embodiment of the invention, at the end of each conductor is attached a cable loop preferably programmed with nano frequencies to complete the circuit.

Typically, the shielding device according to the present invention is provided in a suitable casing. Such casing might be made of stone salt, stone or minerals, glass, wood, plastics or metal, and may have a window for looking into the interior of the shielding device. The door of the casing box might have e.g. 25 to more than 200 perforation holes of a quarter to half a cm width depending on the capacity of units to allow for air ventilation. The window may have a screen and an illumination may be provided.

As already mentioned above, the purpose of the present invention is to harmonise and balance and make less harmful and more biologically friendly to humans, animals and plants the strong electromagnetic frequencies generated by the substantial electricity usage in modern homes, offices and work places and where high electricity is used to power light, machinery and for locomotion. As a result of the effect of the shielding device in accordance with the present invention, the electrons and magnetic frequencies are altered and are more efficiently channeled and absorbed through the electricity cables in the system and the positive effects are then channeled throughout the whole building electrical system.

### PROOF OF EFFECTIVENESS OF THE TECHNOLOGIES OF THE PRESENT INVENTION

By applying worldwide well known Japanese inventor Dr. Masaru Emoto crystal analysis of energy frequencies, the inventor has carried out a crystal analysis of the frequencies coming out of the complete device with the Shen-Ion HarmoNIK programmed plate included. The result is one sees beautiful and well formed crystals with the centre of the crystals forming new life forms meaning this system has very positive biological effects on humans, animals and plants when it is installed in a building or a location that use electricity.

In principle, a preferred shielding device shall consist of at least one borosilicate glass tube of diameter from 0.25 cm to three meter of various lengths and thicknesses according to electricity consumption requirement for each project or installation. Copper wire of different sizes according to the requirement of each project are wrapped around by fibreglass and put inside the glass tube to complete one set of conduction tube. Each of the complete frequency attenuation unit is preferably programmed with nano frequencies among many purposes is to harmonise, calming down and alter the electromagnetic radiation frequencies to be more biologically friendly.

The outer sides around the tube shall be painted with "S" symbols vertically or horizontally or a coat of graphite or metallic base paints with good conduction and deflection can be painted over. A basic unit shall consist of one live borosilicate conduction glass tube or metallic tube as per requirement together with a neutral conduction tube. Larger units can consist of 50 pieces of borosilicate glass or metallic tubes of different sizes according to requirements.

In a preferred embodiment of the invention, an important part is the multiple nano frequencies programmed information in the Shen-Ion HarmoNIK(TM) metal plate or any type of metal plate according to the requirement of each specific project. The programmed frequencies are considered to radiate and to pulse balancing, harmonious, grounding, revitalisation and reprogrammed aggressive frequencies to calm them down to become more human, animal and plants user friendly throughout the electrical system in a building or a location where electricity is used.

The Shen-Ion HarmoNIK(TM) metal plate is also programmed with multiple programs to attract, absorb and suck free electrons, tachyons and static electricity from the air and also resulting in saving of electricity consumption.

The design of box to house the shielding device can be a square, round, octagon, 5, 6, 8 sided or any dimensions for marketing purposes. The complete shielding device can be just plugged into a house, office, ship, aircraft or any electricity utility socket to commence operation. For larger electricity consumption facilities, shielding devices can be connected to the in-house fuse box cables.

Further features and advantages of the present invention may be seen from the description of an example, and by means of the figures.
- Fig. 1: is a schematic perspective view of a shielding device in accordance with the present invention;
- Fig. 2: is a cross sectional view of a frequency attenuation unit as used in a shielding device of the present invention;
- Fig. 3: is a perspective view of a preferred Shen-Ion HarmoNIK (TM) metal plate;
- Fig. 4: shows crystals formed according to Emoto of distilled water samples which were exposed to the shielding device of the present invention; and
- Fig. 5: shows the usual non-crystal forming of distilled water which has not been exposed to the shielding device of the present invention.

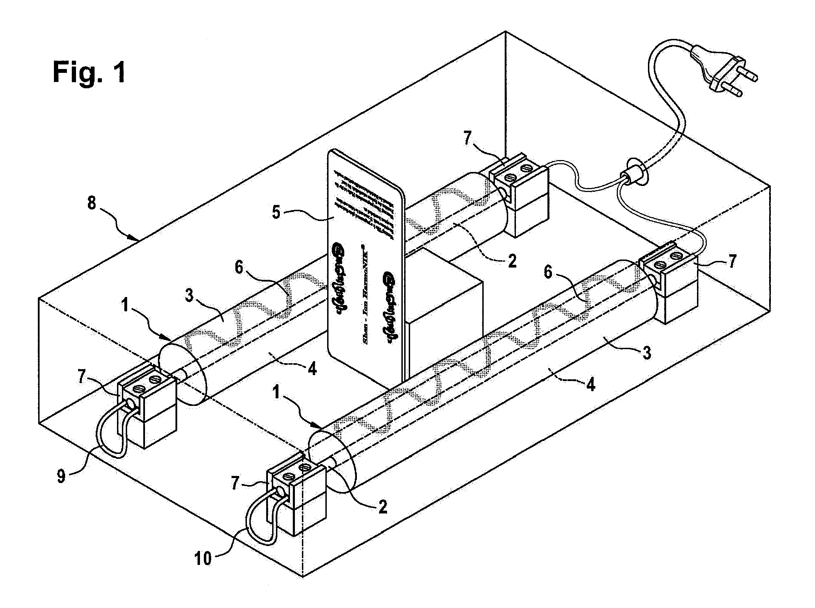

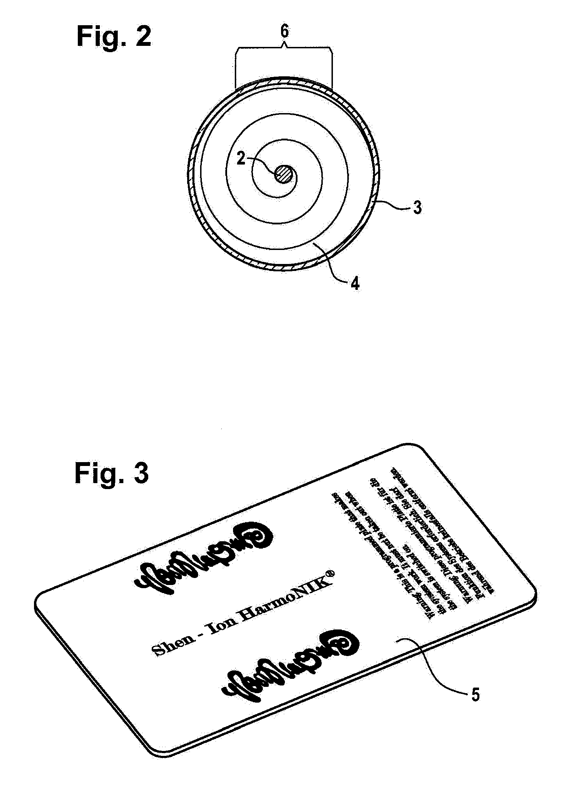

In Fig. 1 a shielding device according to the present invention is shown. For operation, the shielding device for attenuating electromagnetic fields in buildings is connected to an AC power source which is not shown in Fig. 1. It comprises in the present exemplified embodiment two frequency attenuation units 1, each comprising a copper conductor 2, being coaxially arranged in a borosilicate glass tube 3, wherein said copper conductor 2 is wrapped in woven a glass fibre mat as an insulating material 4 (see Fig. 2), and said copper conductor 2 is electrically connected to said power source. Each of the two frequency attenuation units are then programmed with nano frequencies to harmonise, calming down and alter the electromagnetic frequencies to be more biologically friendly. Furthermore, in the shielding device according to the example, one metal plate 5 made of pure aluminum, is provided between the two frequency units 1. In a particularly preferred embodiment of the invention, the metal plate 5 is a Shen-Ion HarmoNIK(TM) aluminum plate (see Fig. 3) which is also programmed with multiple programs to harmonise and alter electromagnetic frequencies and to attract, absorb and suck free electrons, tachyons and static electricity from the air.

For an enhancement of the attenuation effectiveness for EMFs, the borosilicate glass tubes 3 have vertically arranged S-shaped patterns 6 with metal containing paint on their surfaces.

As may be seen from Fig. 1, such parts of the conductors 2 which are located outside the frequency attenuation units 1, are supported by cradles 7. A cable loop programmed with nano frequencies is attached to the end of each of the conductors which are marked 9 and 10 to complete the circuit.

Finally, the shielding device is positioned within an in Fig. 1 front side opened aluminum casing 8.

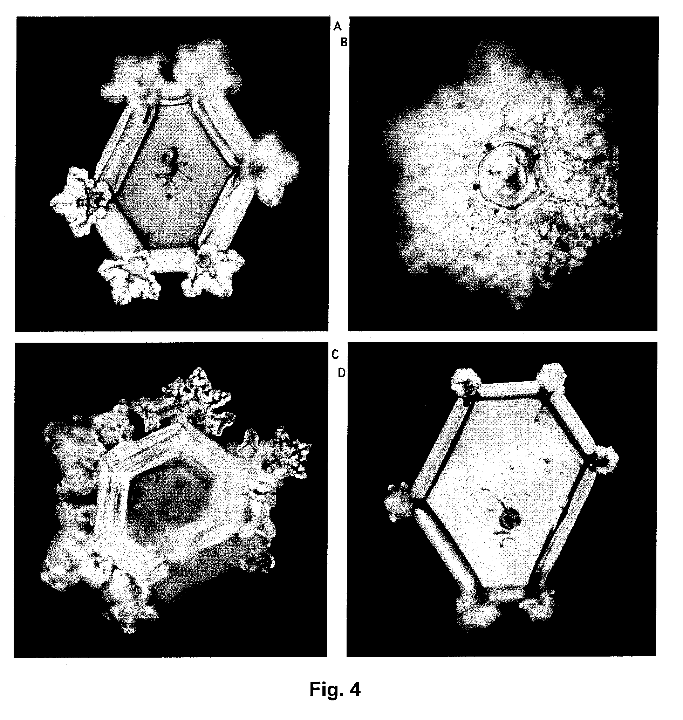

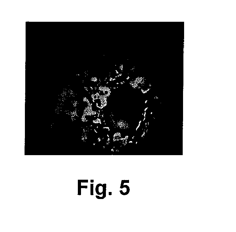

Fig. 4 shows crystals of distilled water samples, in accordance with the above described method by Emoto. Distilled water samples were placed for 5 minutes in close vicinity of the shielding device in accordance with the present invention. A number of drops with an approximate volume of 50 to 100 µl were then frozen at -30°C for 1 h and crystals formed at the tips of the pyramidal shaped frozen blocks were observed and photographed under an optical microscope at a magnification of appr. 20X. Typically, distilled water only in rare cases forms crystals under the above given conditions (cf. Fig. 5). However, as may be seen from Fig. 4, pictures A to D, beautiful and sharply detailed crystals are formed when water was exposed to the shielding device of the present invention and then subjected to the above described Emoto method. Consequently, the shielding device of the present invention is able to change the energetic properties of the water, which is indicative of the shielding effect of the present invention's device.

## Claims

1. Shielding device for attenuating electromagnetic fields in buildings **characterized by** comprising:
a) an AC power source;
b) at least two frequency attenuation units (1), each comprising a conductor (2), being coaxially arranged in a glass or metal tube (3), wherein said conductor (2) is wrapped in an insulating material (4), and said conductor (2) is electrically connected to said power source; and
c) at least one metal plate (5), provided between two of said frequency units (1).

2. Shielding device according to claim 1, **characterized in that** said conductors (2) are selected from the group consisting of: graphite, aluminium, titanium, vanadium, chromium, molybdenum, iron, cobalt, nickel, zinc, copper, silver, gold, platinum, palladium.

3. Shielding device according to claim 1 or 2, **characterized in that** said conductors (2) are made of copper.

4. Shielding device according to anyone of claims 1 to 3, **characterized in that** said glass tubes (3) are selected from the group consisting of: alkali silicate glass, earth alkaline glass, quartz, borosilicate glass.

5. Shielding device according to anyone of claims 1 to 4, **characterized in that** said glass tubes (3) are made of borosilicate glass.

6. Shielding device according to anyone of claims 1 to 3, **characterized in that** said metal tube (3) is made of a metal being selected from the group consisting of: aluminium, titanium, vanadium, chromium, molybdenum, iron, cobalt, nickel, zinc, copper, silver, gold, platinum, palladium; and alloys thereof.

7. Shielding device according to anyone of claims 1 to 6, **characterized in that** said insulating material (4) is a bound or unbound, woven or unwoven mineral wool or glass fibre wool, in particular a woven glass fibre mat.

8. Shielding device according to anyone of claims 1 to 7, **characterized in that** said glass tubes (3) of said frequency attenuation units (1) bear vertically or horizontally arranged patterns (6) on their surfaces.

9. Shielding device according to claim 8, **characterized in that** said patterns (6) are S-shaped, in particular, the S-shaped patterns are continuous along the glass or metal tubes (3), or broken up in small sections either horizontally or vertically placed.

10. Shielding device according to claim 8 or 9, **characterized in that** said patterns (6) are created by grooves, or coatings made of graphite or metallic base paints showing a high conductivity and/or deflection properties for electromagnetic fields.

11. Shielding device according to anyone of claims 1 to 10, **characterized in that** said glass tubes (3) of said frequency attenuation units (1) have a diameter of 0.25 cm to 3 m.

12. Shielding device according to anyone of claims 1 to 11, **characterized in that** said metal plate (5) between said frequency attenuation units (1) is made of a metal being selected from the group consisting of: aluminium, titanium, vanadium, chromium, molybdenum, iron, cobalt, nickel, zinc, copper, silver, gold, platinum, iridium, palladium; and alloys thereof.

13. Shielding device according to claim 12, **characterized in that** said metal plate (5) is made of aluminium.

14. Shielding device according to claim 13, **characterized in that** said aluminium plate (5) is provided for absorbing free electrons, tachyons and static electricity from the air.

15. Shielding device according to anyone of claims 1 to 14, **characterized in that** such parts of said conductors (2) which are located outside the frequency attenuation units (1), are supported by at least one cradle (7).

16. Shielding device according to anyone of claims 1 to 15, **characterized in that** the device is provided in a casing (8).

17. Shielding device according to anyone of claims 1 to 16, **characterized in that** a door is provided at the casing of the device, wherein said casing door is perforated for air ventilation, preferably with 25 to 200 holes having a diameter of at least appr. 0,25 cm.
